(19) Europäisches Patentamt
European Patent Office
Office européen des brevets

(11) EP 2 218 465 A1

(12) EUROPÄISCHE PATENTANMELDUNG

(43) Veröffentlichungstag:
18.08.2010  Patentblatt 2010/33

(51) Int Cl.:
A61L 2/08 (2006.01)    B65B 55/08 (2006.01)

(21) Anmeldenummer: 10000337.5

(22) Anmeldetag: 15.01.2010

(84) Benannte Vertragsstaaten:
AT BE BG CH CY CZ DE DK EE ES FI FR GB GR
HR HU IE IS IT LI LT LU LV MC MK MT NL NO PL
PT RO SE SI SK SM TR
Benannte Erstreckungsstaaten:
AL BA RS

(30) Priorität: 02.02.2009  DE 102009007112

(71) Anmelder: KHS GmbH
Juchostrasse 20
44143 Dortmund (DE)

(72) Erfinder: Keil, Gernot, Dr.
55595 Braunweiler (DE)

(54) **Vorrichtung zum Sterilisieren eines Behälters**

(57)  Die vorliegende Erfindung betrifft eine Vorrichtung (100) zum Sterilisieren eines Behälters (30), insbesondere einer Flasche, mit mindestens einem Elektronenstrahlerzeuger (10) zum Erzeugen eines Elektronenstrahlbündels (12) zum Sterilisieren des Behälters (30) und mindestens einer Fokussiereinrichtung (20) zum Fokussieren des Elektronenstrahlbündels (12), so dass das Elektronenstrahlbündel (12) fokussiert durch eine Behälteröffnung (32) in den Behälter (30) eintritt. Des weiteren betrifft die vorliegende Erfindung ein Verfahren zum Sterilisieren eines Behälters (30).

Fig. 1

EP 2 218 465 A1

**Beschreibung**

**Technisches Gebiet**

Die vorliegende Erfindung betrifft eine Vorrichtung zum Sterilisieren eines Behälters, insbesondere einer Flasche, und ein Verfahren zum Sterilisieren eines Behälters.

**Beschreibung des Standes der Technik**

Bei der Verpackung von Lebensmitteln und bei dem Abfüllen von Getränken in Behälter oder Flaschen besteht eine der Hauptanforderungen darin, die Lebensmittel bzw. Getränke keimfrei zu verpacken bzw. abzufüllen. Dafür ist es insbesondere wichtig, dass die Verpackungsmittel und Flaschen steril sind.

Zur Sterilisation von Stoffen und Vorrichtungen sind verschiedene Verfahren bekannt. Insbesondere wurde bereits vorgeschlagen, Strahlung zur Sterilisation zu verwenden. Neben infraroter, ultravioletter und MikrowellenStrahlung wurde auch vorgeschlagen, Elektronenstrahlen zur Sterilisation einzusetzen.

Eine entsprechende Vorrichtung zur Oberflächensterilisation von Stoffen ist in dem deutschen Patent DE 22 27 059 C3 beschrieben.

Auch auf dem Anwendungsgebiet der Getränkeabfüllung, mit dem sich die vorliegende Erfindung vorrangig, aber nicht ausschließlich beschäftigt, wurden Elektronenstrahlen bereits zur Sterilisation der Flaschen und Verschlüsse vorgeschlagen.

Eine entsprechende Vorrichtung und ein Verfahren zum Sterilisieren und Füllen von Flaschen sind in der Druckschrift US 2007/0237672 A1 beschrieben. Die dort beschriebene Vorrichtung umfasst mehrere Fördereinrichtungen, die als umlaufende Karussells ausgebildet sind und Flaschen in einer Abfüllanlage fördern. In der Abfüllanlage ist eine Station vorgesehen, in der die Flaschen sterilisiert werden.

Eine Schwierigkeit hierbei liegt in der Regel darin, dass die Flaschen nicht nur äußerlich, sondern auch an ihrer Innenseite sterilisiert werden müssen, da hierzu der entsprechende Elektronenstrahl durch die verhältnismäßig kleine Flaschenöffnung in das Flascheninnere gelangen und sich dort möglichst gleichmäßig verteilen muss.

In der US 2007/0237672 A1 wird daher vorgeschlagen, den Elektronenstrahl in einem Winkel zur Längsachse der Flasche in die Flasche zu leiten, so dass der Elektronenstrahl durch mehrfache Reflexion an der Innenseite der Flasche den Innenraum auf seiner gesamten Länge durchwandert. Des weiteren wird vorgeschlagen, die Flasche um ihre Längsachse zu rotieren oder aber die Elektronenkanone zu bewegen, um den gesamten Winkelbereich der Flaschenoberfläche zu sterilisieren.

Die vorgeschlagenen Vorrichtungen weisen jedoch den Nachteil auf, dass die Elektronenstrahlen eine verhältnismäßig große Pfadlänge im Inneren der Flasche zu durchwandern haben, wobei sie auf ihrem Weg mehrfach reflektiert werden. Die Elektronenstrahlen müssen daher eine relativ hohe Reichweite aufweisen, damit sie den vorgesehenen Pfad vollständig abwandern. Des weiteren sind Einrichtungen vorzusehen, um die Flasche oder die Elektronenkanone zu bewegen, um den gesamten Winkelbereich der Flasche zu bestreichen.

**Zusammenfassung der Erfindung**

Demgegenüber wird erfindungsgemäß eine Vorrichtung zum Sterilisieren eines Behälters nach Anspruch 1 und ein Verfahren zum Sterilisieren eines Behälters nach Anspruch 13 bereitgestellt.

Die Vorrichtung zum Sterilisieren eines Behälters, insbesondere einer Flasche, umfasst mindestens einen Elektronenstrahlerzeuger zum Erzeugen eines Elektronenstrahlbündels zum Sterilisieren des Behälters und mindestens eine Fokussiereinrichtung zum Fokussieren des Elektronenstrahlbündels, so dass zumindest ein Teil, vorzugsweise der überwiegende Teil des Elektronenstrahlbündels fokussiert durch eine Behälteröffnung in den Behälter eintritt. Durch Fokussierung des Elektronenstrahlbündels, das aus dem mindestens einen Elektronenstrahlerzeuger, bspw. einer Elektronenröhre, in parallelen oder leicht divergenten Strahlen austritt, auf einen kleinen Raumbereich, der unmittelbar vor der Flaschenöffnung, im Halsbereich der Flasche oder im oberen inneren Bereich der Flasche liegt, kann die gesamte innere Flaschenoberfläche mit Elektronen bestrahlt werden. Durch die Fokussierung wird erreicht, dass das Strahlenbündel im Bereich des Fokus eine geringe Flächenausdehnung hat und somit leicht durch den Flaschenhals treten kann. Auf diese Art kann ein größerer Elektronenstrom, der durch eine Elektronenröhre bereitgestellt wird, zu Desinfektionszwecken an der Flasche genutzt werden. Des weiteren fächert sich das Strahlenbündel jenseits des Fokuspunktes wieder auf und verteilt sich in dem gesamten Flascheninnenraum, ohne dass bereits mannigfaltige Strahlenanteile einmal reflektiert worden sind, d.h. auf direktem Weg.

Durch die Fokussierung und den Energieverlust der Elektronen beim Durchgang durch Absorbermaterial bildet sich am Ende der Reichweite der Elektronenstrahlen eine diffuse Elektronenwolke, die Kontakt mit der Innenseite des Behälters hat. Durch Variation der Elektronenenergie, der Spannung oder des Magnetfeldes einer Elektronenröhre kann die Form und die relative Höhe der Elektronenwolke beeinflusst werden. Die Reichweite der Elektronen in Luft nimmt rasch mit zunehmender Röhrenspannung zu. Für Röhrenspannungen von 100 kV, 150 kV, 200 kV und 300 kV erhält man bspw. Reichweiten von etwa 16 mg/cm$^2$, 32 mg/cm$^2$, 51 mg/cm$^2$ bzw. 95 mg/cm$^2$, wobei der Wert von 95 mg/cm$^2$ einer Reichweite der Elektronen in trockener Luft und unter Normaldruck (1013 hPa, 20°C) von 80 cm entspricht, wenn man unter der Reichweite den Anfang der Bahn bis zum weitesten Punkt der Bahn versteht und unter dem Anfang der Bahn den Austrittspunkt eines Elektrons aus dem Elektronenstrahlerzeuger.

Die Reichweite des Elektronenstrahls bzw. die Einheit [mg/cm$^2$] wird dabei als "durchstrahlte Masse" angegeben. Die angegebene Fläche wird auf 1 cm$^2$ bezogen. Ein Elektronenstrahl mit der Querschnittsfläche von 1 cm$^2$ und einer Reichweite von 32 mg/cm$^2$ dringt bspw. so weit bzw. tief in einen Stoff ein, bis dass er eine Masse von 32 mg durchstrahlt hat.

Für Magnetfelder zwischen einigen hundert Gauß bis zu etwa einem Tesla lassen sich Fokusabstände zwischen etwa einem Zentimeter und einigen hundert Zentimetern erzeugen, wenn die Elektronenenergie dabei zwischen 100 und 300 kV liegt.

Die Reichweite der Elektronen in der Luft oder auch in anderen Absorbern kann durch die Variation der Spannung der Elektronenröhre verändert werden. Es ist bekannt, dass Elektronenstrahlen durch Abbremsung in Luft zunehmend Energie verlieren und diffuser werden. Ein anfänglich paralleler Elektronenstrahl, der eine Anfangsenergie Ea besitzt, wird in den ersten 50 % seiner Reichweite kaum aufgefächert, wobei mit der Anfangsenergie die Energie der Elektronen direkt nach dem Austritt aus der Elektronenröhre bezeichnet ist. Hat der Strahl jedoch genügend Energie durch Stöße mit dem Absorber erlitten, beginnt er aufzufächern. Verliert ein Strahl 95 % seiner Energie, dann wird er stark divergent und mit weiterem Energieverlust sogar diffus. Die diffusen Elektronen besitzen aber immer noch Energien von etwa 100 bis 5000 eV und sind damit auch noch mikrobizid, da sie noch Molekülbindungen lösen können.

Durch die Variation der Elektronenenergie Ea kann somit der Raumbereich, in dem der Elektronenstrahl diffus wird, in der Flasche verschoben werden. Dazu kann beispielsweise die Röhrenspannung sägezahnartig zeitlich variiert werden. Dabei sind Spannungshöhen von 0,2 Umax bis 1,0 U-max der maximalen Elektronenstrahlspannung Umax in einer Zeit von weniger als 0,5 s erreichbar.

Wird zusätzlich zur Variation der Elektronenstrahlspannung der Fokuspunkt der Fokussiereinrichtung in geeigneter Weise verändert, dann erreicht man eine günstige Injektion des Elektronenstrahles in die Flasche.

Durch die axial aufeinanderfolgende Anordnung des mindestens einen Elektronenstrahlerzeugers, der mindestens einen Fokussiereinrichtung und des entsprechenden Behälters wird des weiteren vermieden, den Elektronenstrahlerzeuger in die Flasche eintauchen zu müssen.

Es kann vorgesehen sein, dass die Fokussiereinrichtung ein Permanentmagnet ist. Es kann aber auch vorgesehen sein, dass die Fokussiereinrichtung ein fremdinitiierter Elektromagnet ist. Dabei kann der Strom des fremdinitiierten Elektromagneten variierbar sein, um unterschiedliche Zonen einer Innenseite des Behälters mit dem Elektronenstrahlbündel zu bestrahlen. Es kann also bspw. vorgesehen sein, die Einstellungen des Elektronenstrahlerzeugers unverändert zu lassen und lediglich durch Variation des Stroms des als Fokussiereinrichtung dienenden Elektromagneten den Ort des Fokus und damit den Verlauf des Elektronenstrahlbündels zu variieren.

Ist als Fokussiereinrichtung ein Permanentmagnet vorgesehen, kann dieser scheibenförmig ausgestaltet sein und ein Loch mit geeignetem Durchmesser aufweisen, wobei die Magnetisierung derart ausgestaltet ist, dass in dem Loch ein axiales Magnetfeld erzeugt wird. Zum Schutz kann der Permanentmagnet an seinen Außenflächen mit einer Metallisierung versehen sein.

Wenn als Fokussiereinrichtung ein fremdinitiierter Elektromagnet vorgesehen ist, kann dieser als Solenoid, d.h. als Zylinderspule, ausgestaltet sein. Das Solenoid kann mit einer Flussrückführung, die die Oberfläche des Solenoids mindestens teilweise bedeckt, versehen sein.

Selbstverständlich kann ergänzend oder alternativ auch vorgesehen sein, dass sich der Behälter bzw. die Flasche "auf und ab" bewegt, d.h. parallel zu einer Längsachse des Elektronenstrahlerzeugers und einer Längsachse der Fokussiereinrichtung. Dabei kann der Fokus der Elektronenstrahlen ortsfest verbleiben, und der Behälter bzw. die Flasche wird relativ zu dem Fokus bewegt.

Selbstverständlich können die drei voranstehend beschriebenen Maßnahmen zur Variation der relativen Position des Elektronenstrahlfokus und des Behälters zueinander sowohl alternativ als auch kumulativ vorgesehen sein.

Des weiteren kann vorgesehen sein, dass die erfindungsgemäße Vorrichtung eine Fördereinrichtung zum Fördern des Behälters umfasst. Diese Fördereinrichtung kann ein umlaufendes Karussell sein.

Die relative Bewegung von dem mindestens einen Elektronenstrahlerzeuger, der mindestens einen Fokussiereinrichtung und des Behälters zueinander kann dabei unterschiedlich ausgelegt sein.

So kann bspw. vorgesehen sein, dass jeder Flasche auf dem umlaufenden Karussell eine Fokussiereinrichtung und ein Elektronenstrahlerzeuger zugeordnet ist, die die Flasche sterilisieren, während sie sich auf dem entsprechenden Karussell befindet. Jeder möglichen Flaschenposition auf dem Karussell wird entsprechend ein Elektronenstrahlerzeuger und eine Fokussiereinrichtung zugeordnet. Ein Vorteil ergibt sich hierbei insbesondere dadurch, dass eine Sterilisation des Behälters bzw. der Flasche während seiner gesamten Verweildauer auf dem Karussell möglich ist.

Es kann aber auch vorgesehen sein, dass nur ein ortsfester Elektronenstrahlerzeuger vorgesehen ist, und sich die Behälter bzw. Flaschen mit jeweils einer ihnen zugeordneten Fokussiereinrichtung unter dem Elektronenstrahlerzeuger hindurchbewegen. An einer entsprechenden Stelle wird dann ein Elektronenstrahlimpuls in die Flaschen gestrahlt. Bei dieser Anordnung ist entsprechend nur ein Elektronenstrahlerzeuger vorzusehen, wobei jedoch die Dauer der Sterilisation von der Verweildauer des Behälters bzw. der Flasche unter dem einen Elektronenstrahlerzeuger abhängt.

Weitere Vorteile und Ausgestaltungen der Erfindung ergeben sich aus der Beschreibung und der beiliegenden Zeichnung.

Es versteht sich, dass die voranstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

## Kurzbeschreibung der Zeichnung

Figur 1 zeigt eine erfindungsgemäße Vorrichtung in einer Ausführungsform.

Figur 2 zeigt eine erfindungsgemäße Vorrichtung in einer weiteren Ausführungsform.

Figur 3 zeigt die erfindungsgemäße Vorrichtung in der weiteren Ausführungsform, wobei der Fokus des Elektronenstrahlbündels gegenüber der Darstellung in Figur 2 verschoben ist.

Figur 4 zeigt schematisch die Ausbreitung eines Elektronenstrahls mit einer Elektronenenergie Ea.

Figur 5 zeigt schematisch die Ausbreitung eines Elektronenstrahls mit einer Elektronenenergie von 1,5 Ea.

Figur 6 zeigt schematisch eine Quadrapolanordnung einer Fokussiereinrichtung.

Figur 7 zeigt schematisch eine Sextupolanordnung einer weiteren Fokussiereinrichtung.

## Ausführliche Beschreibung

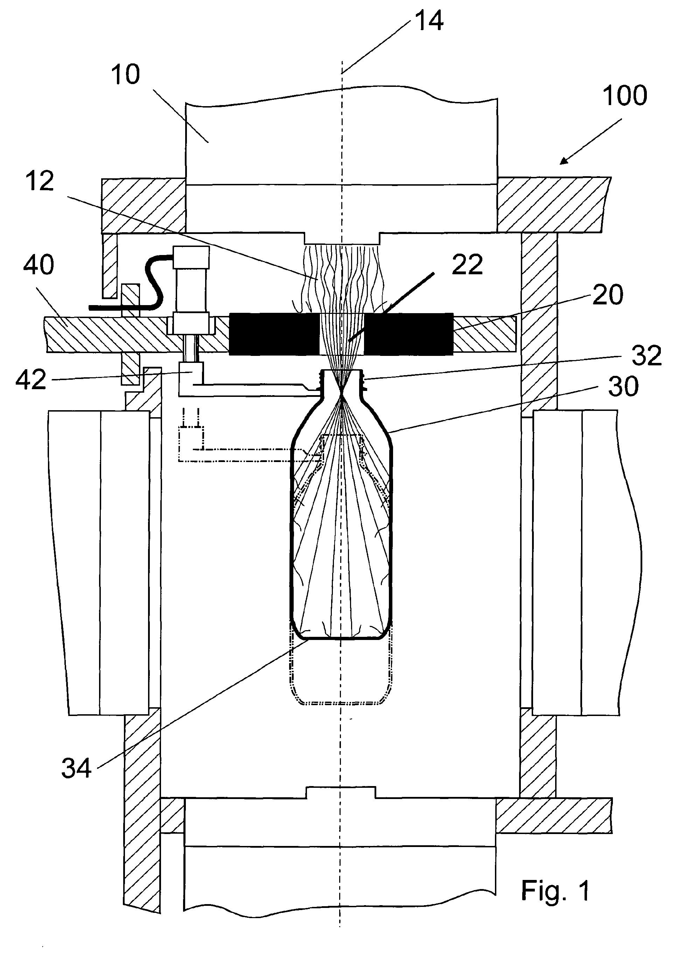

Figur 1 zeigt eine erfindungsgemäße Vorrichtung 100 zum Sterilisieren eines Behälters 30, der in der dargestellten Ausführungsform eine Flasche ist.

Die Flasche 30 wird von einer Fördervorrichtung 40, die als umlaufendes Karussell ausgebildet ist, unter eine Elektronenröhre 10 bewegt.

Eine Fokussiereinrichtung 20 ist dabei der Flasche 30 fest zugeordnet, wobei die Flasche mittels eines Arms 42 axial fluchtend zu einer Öffnung 22 im Zentrum der Fokussiereinrichtung 20 auf der der Elektronenröhre 10 abgewandten Seite der Fokussiereinrichtung 20 angeordnet ist.

Ein von der Elektronenröhre 10 erzeugtes Elektronenstrahlbündel 12 trifft daher zunächst auf die Fokussiereinrichtung 20, die in der dargestellten Ausführungsform bspw. ein Permanentmagnet ist. Die Position des Arms 42 und damit der Flasche 30 relativ zu den Permanentmagneten 20 und die Stärke des Permanentmagneten ist dabei so ausgewählt, dass der Fokus in einem Hals 32 der Flasche 30 liegt. Auf diese Weise treten sämtliche, durch die Öffnung 22 des Permanentmagneten 20 tretenden Elektronenstrahlen in die Flasche 30 ein. Des weiteren fächert sich das Elektronenstrahlbündel 12 jenseits des Fokus wieder auf, so dass die gesamte Innenseite 34 der Flasche 30 mit Elektronen bestrahlt wird.

Bei anderen Flaschenformen und -längen kann der Arm 42 relativ zu der Fokussiereinrichtung 20 axial verschoben werden, so dass eine Anpassung an individuelle Flaschengrößen und Formen vorgenommen werden kann.

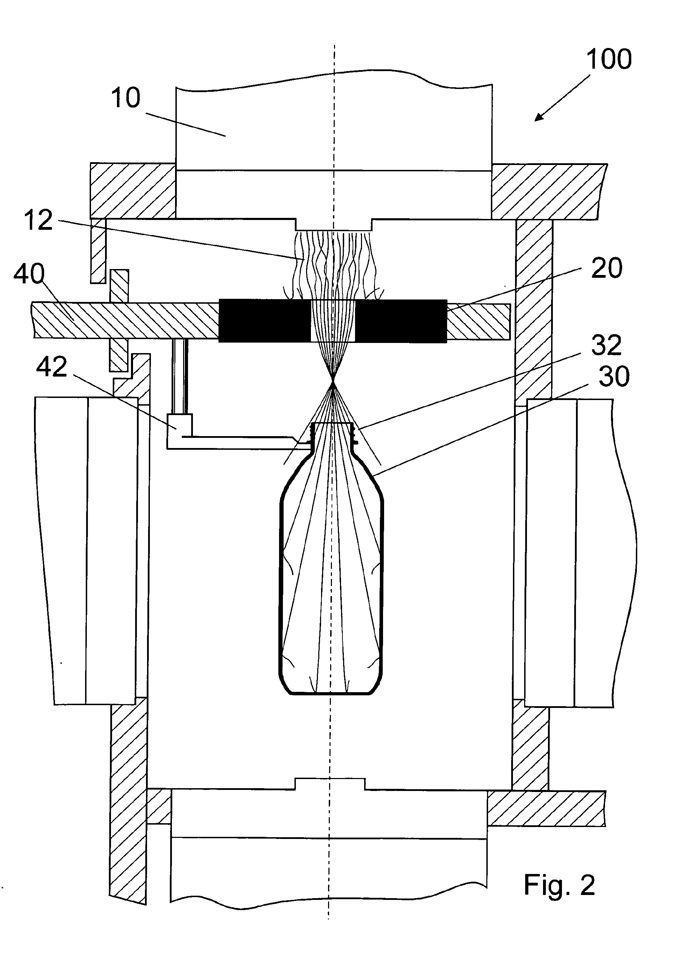

Die in Figur 2 dargestellte Ausführungsform entspricht im wesentlichen der in Figur 1 dargestellten Ausführungsform, der Arm 42 ist jedoch nicht relativ zu der Fokussiereinrichtung 20 axial verschiebbar, so dass keine Anpassung an verschiedene Flaschengrößen und -formen durch ein Verschieben des Arms 42 vorgenommen werden kann.

Somit kann es vorkommen, dass der Fokus nicht mehr im Hals 32 der Flasche 30 liegt, sondern wesentlich davor, so dass einige Elektronen nicht in den Innenraum der Flasche 30 eintreten.

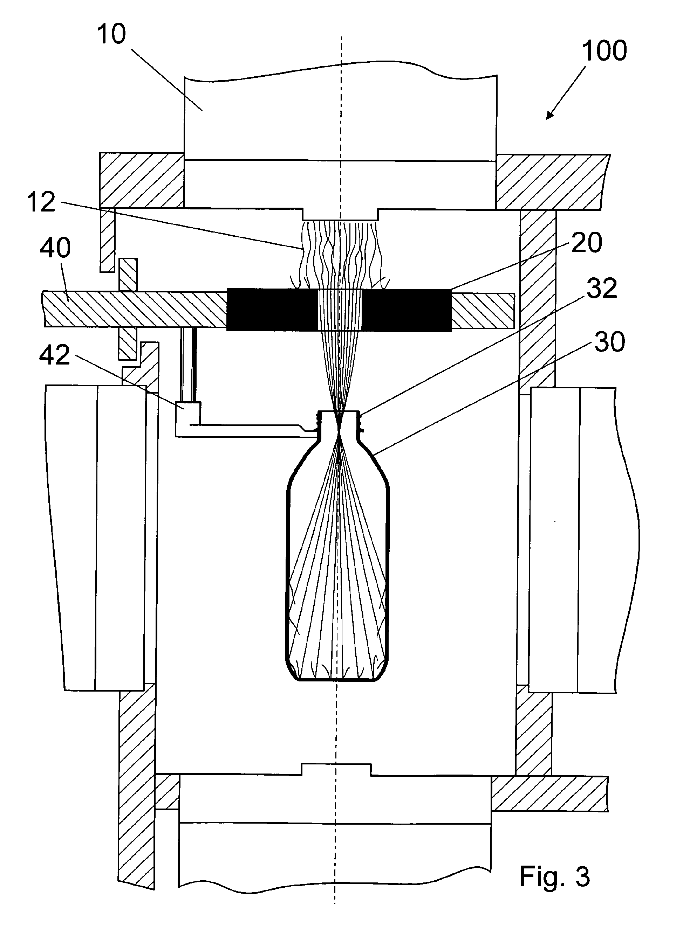

Entsprechend ist vorgesehen, dass eine Spannung, eine Elektronenenergie oder ein Magnetfeld der Elektronenröhre 10 variierbar ist und/oder dass als Fokussiereinrichtung 20 kein Permanentmagnet, sondern eine Zylinderspule vorgesehen ist, deren Magnetfeld durch die Veränderung von Spannung und/oder Strom variierbar ist. Auf diese Weise lässt sich der Fokus des Elektronenstrahlbündels 12 verändern und so einstellen, dass er im Hals 32 der Flasche 30 liegt, wie in Figur 3 dargestellt ist.

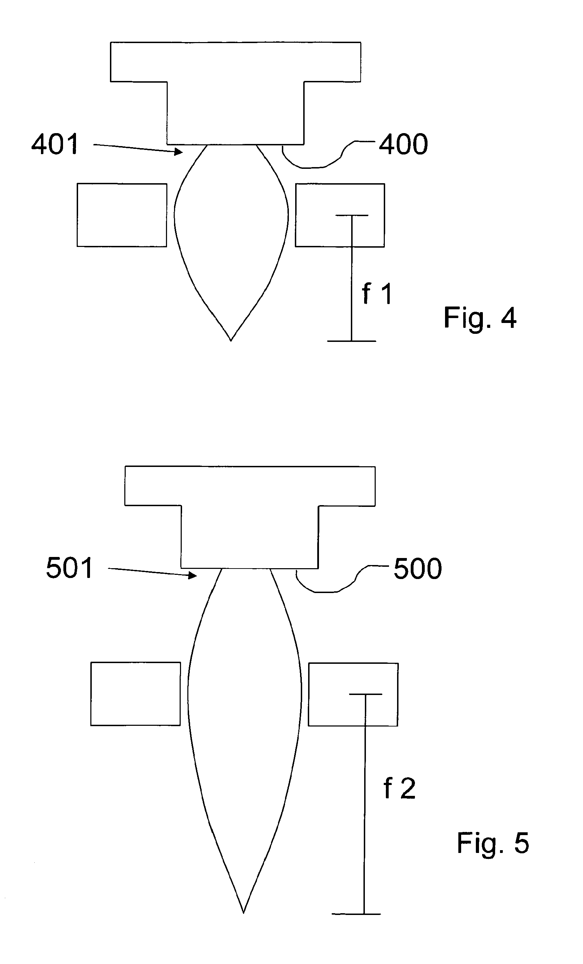

Figur 4 zeigt schematisch den Verlauf von Elektronen 401, die in einem Elektronenaustrittsfenster 400 eine Elektronenenergie Ea aufweisen. Mittels einer Fokussiereinrichtung 402 wird eine erste Fokallänge f1 erreicht.

Grundsätzlich ist es möglich, die Fokallänge lediglich durch Variation der Elektronenenergie zu variieren. So zeigt Figur 5 schematisch den Verlauf von Elektronen 501, die in einem Elektronenaustrittsfenster 500 eine Elektronenenergie von 1,5 Ea aufweisen. Mittels einer Fokussiereinrichtung 502, die der in Figur 4 entspricht, wird so eine zweite Fokallänge f2 erreicht, die größer als die erste Fokallänge f1 ist.

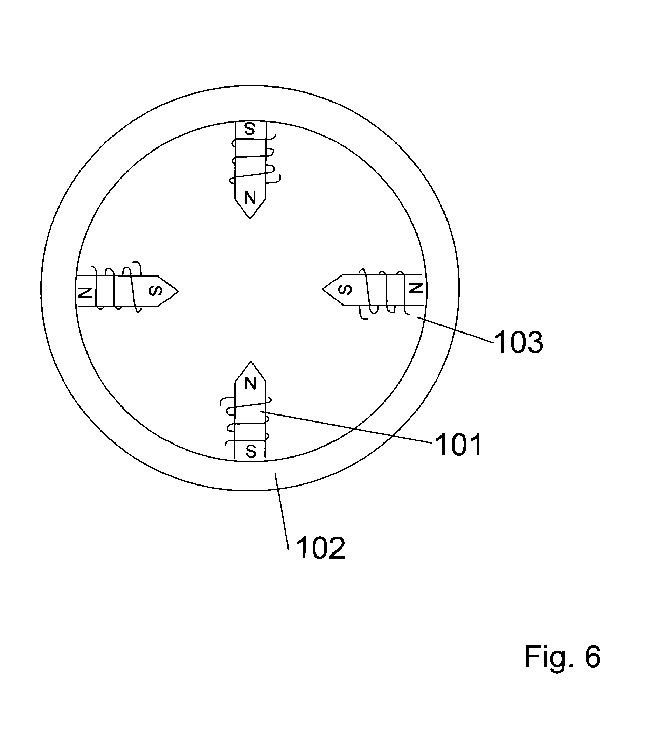

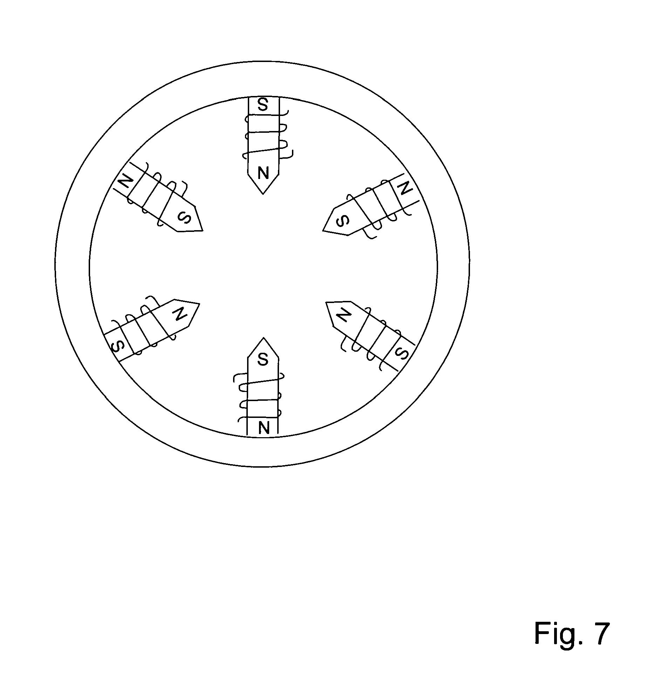

Als Fokussierelemente können Multipolfelder mit Feldcharakteristiken vom Typ Quadrupolfeld, Sextupolfeld, Oktupolfelder, usw. herangezogen werden. Jede Charakteristik mit einer Anzahl von n Polpaaren, wobei $n>=1$ gilt, ist grundsätzlich möglich. Ein Dipolfeld ($n=1$) besitzt jedoch keinen Vorteil, weil es genauso viele Elektronen fokussiert wie defokussiert. Insgesamt gute Eigenschaften weist eine Sextupolanordnung auf, da sie eine gute Fokussierung mit einem konstruktiv einfachen Aufbau verbindet. Eine Anordnung eines magnetischen Quadrupols ist in Figur 6 dargestellt. Eine Anordnung eines magnetischen Sextupols ist in Figur 7 dargestellt. Besonders vorteilhaft an der Verwendung eines Quadrupols oder eines Sextupols ist, dass sich verbesserte Möglichkeiten zur Steuerung des Elektronenstrahls ergeben. So ist es beispielsweise möglich, den Elektronenstrahl so zu steuern, dass er sich in der Zeichnungsebene der Figuren 6, bzw. 7 bewegen würde.

Ein Quadrupol wird zum Beispiels durch die Anordnung von 4 magnetisierbaren Polschuhen 101 in einem magnetisierbaren Joch 102 und durch die Erzeugung von Magnetfeldern, angedeutet durch die Pole "N" und "S" in den Figuren 6 und 7 generiert. In der Literatur werden auch n-Pole beschrieben, die aus einzelnen Permanentmagneten aufgebaut sind.

Die Fokallänge der Axialmagnetfelder wird durch deren Feldverlauf bestimmt. Die Fokallänge kann beispielsweise für eine kurze Axialspule anhand der Busch schen Brennweiten-Formel

$$\frac{1}{f} = \frac{1}{4}\left(\frac{e}{mv}\right)^2 \int B_z^2\,(z)\ dz,$$

berechnet werden, wobei e die Elementarladung, m die Masse des Elektrons, v die Geschwindigkeit des Elektrons, $B_z$ die magnetische Induktion in Ausbreitungsrichtung des Elektrons und f die Fokallänge ist. Das Intergral $\int B_z^2(z)$ dz beträgt typischerweise $1*10^8$ $A^2$/m für eine kurze Spule, d.h. für eine Spule, deren mittlerer Durchmesser mindestens 1,5 mal größer ist als ihre Länge in axialer Richtung ist, für eine Feldstärke im Maximum von 1000 Oe, wobei die Maximalfeldstärke durch den Spulenstrom einstellbar ist.

Die Fokallänge ist die Distanz zwischen Spulenmitte und Fokuspunkt.

Die nachstehende Tabelle listet die anhand der Busch'schen Formel berechneten Fokallängen für Elektronenenergien zwischen 50 keV und 400 keV für Feldintegrale von $1* 10^8$ $A^2$/m auf. Noch kürzere Fokallängen können durch Erhöhung der Feldstärke erzielt werden, was zur Erhöhung des Feldintegrals führt.

| E [keV] | 1/f[1/m] | f [m] |
|---|---|---|
| 50 | 6,60E+01 | 1,52E-02 |
| 100 | 3,15E+01 | 3,17E-02 |
| 150 | 2,01 E+01 | 4,97E-02 |
| 200 | 1,45+01 | 6,91E-02 |
| 250 | 1,11E+01 | 8,99E-02 |

(fortgesetzt)

| E [keV] | 1/f[1/m] | f [m] |
|---|---|---|
| 300 | 8,92E+00 | 1,12E-01 |
| 350 | 7,37E+00 | 1,36E-01 |
| 400 | 6,22E+00 | 1,61E-01 |

Kritisch können hohe Elektronenenergien sein, da sie zu großen Fokallängen führen. Für eine Elektronenenergie von 400 keV kann man mit einer Maximalfeldstärke von 2000 Oe (doppelt so großes Feldintegral wie oben angegeben) in der Mitte der Axialspule eine Fokallänge von 8 cm erreichen. Diese lässt sich noch durch ein Eisenjoch, dass die Feldstärke erhöht, weiter verkürzen, so dass dann Fokallängen von weniger als 4 cm zu erzeugen sind.

Der magnetische Multipol kann etwa durch Permanentmagnete erzeugt werden, wobei dann ein Fokussierungselement mit fixem Fokus vorliegt. Der magnetische Multipol kann aber auch durch Spulen fremderregt werden, siehe Figur 7, wobei dann der Fokuspunkt variabel eingestellt werden kann. Mit Polspitzenfeldern von wenigen 10 Oerstedt (Oe) bis zu Feldern von 2000 Oerstedt deckt man Fokallängen von etwa 1 cm bis etwa 20 cm für Elektronenenergien von 50 bis 400 keV ab.

Der elektrische Multipol wird durch Anlegen von Spannungen im Bereich von etwa 100 bis etwa 5000 Volt (V) an geeignet geformte Elektroden erzeugt, wobei man Fokallängen von etwa 1 cm bis etwa 20 cm erhält. Die durch den elektrischen Multipol erzeugte Fokallänge kann durch Variation der Spannung an den Elektroden variiert werden.

In Elektronenausbreitungsrichtungen haben die Fokussierungselemente des magnetischen Multipols eine Länge von etwa 2 bis etwa 10 cm. Durch Verwendung mehrerer Elemente, die hintereinander angeordnet werden, lässt sich - ähnlich wie in der Optik - eine bessere bzw. angepasste Fokussierung erreichen.

Beim Quadrupolfeld nimmt die Fokussierungskraft proportional mit dem Abstand r zur Symmetrieachse zu. Beim Hexapolfeld (Sechspolfeld) nimmt die Fokussierungskraft quadratisch mit dem Abstand r zur Symmetrieachse zu. Beim Oktupolfeld nimmt die Fokussierungskraft kubisch mit dem Abstand r zur Symmetrieachse zu.

In der voranstehend beschriebenen Ausführungsform ist die Elektronenröhre 10 ortsfest angeordnet. Selbstverständlich kann alternativ auch vorgesehen sein, dass jeder Fokussiereinrichtung 20 eine Elektronenröhre 10 zugeordnet ist, und diese gemeinsam mit einer entsprechenden Flasche 30 auf dem Karussell umlaufen.

**Patentansprüche**

1. Vorrichtung (100) zum Sterilisieren eines Behälters (30), insbesondere einer Flasche, mit:

   mindestens einem Elektronenstrahlerzeuger (10) zum Erzeugen eines Elektronenstrahlbündels (12) zum Sterilisieren des Behälters (30), mindestens einer Fokussiereinrichtung (20) zum Fokussieren des Elektronenstrahlbündels (12), derart dass das Elektronenstrahlbündel (12) fokussiert durch eine Behälteröffnung (32) in den Behälter (30) eintritt.

2. Vorrichtung nach Anspruch 1, die derart ausgestaltet ist, dass sich das Strahlenbündel (12) jenseits eines Fokus der Fokussiereinrichtung (20) wieder auffächert und sich auf direktem Weg in dem gesamten Behälter (30) verteilt.

3. Vorrichtung (100) nach Anspruch 1 oder 2, die des weiteren eine Fördereinrichtung (40) zum Fördern des Behälters umfasst.

4. Vorrichtung (100) nach Anspruch 3, bei der die Fördereinrichtung (40) ein umlaufendes Karussell ist.

5. Vorrichtung (100) nach einem der Ansprüche 1 bis 4, bei der der mindestens eine Elektronenstrahlerzeuger (10) eine Elektronenröhre ist.

6. Vorrichtung (100) nach einem der Ansprüche 1 bis 5, bei der die mindestens eine Fokussiereinrichtung (20) ein Permanentmagnet ist.

7. Vorrichtung (100) nach einem der Ansprüche 1 bis 5, bei der die mindestens eine Fokussiereinrichtung (20) ein fremdinitiierter Elektromagnet ist.

8. Vorrichtung (100) nach einem der Ansprüche 3 bis 7, bei der dem Behälter eine der mindestens einen Fokussiereinrichtung (40) zugeordnet ist, die von der Fördereinrichtung (40) zusammen mit dem Behälter (30) gefördert ist, wobei der Elektronenstrahlerzeuger (10) ortsfest angeordnet ist.

9. Vorrichtung (100) nach einem der Ansprüche 3 bis 7, bei der dem Behälter (30) einer der mindestens einen Elektronenstrahlerzeuger (10) und eine der mindestens einen Fokussiereinrichtung (20) zugeordnet ist, wobei die Fördereinrichtung (40) den zugeordneten Elektronenstrahlerzeuger (10), die zugeordnete Fokussiereinrichtung (20) und den Behälter (30) gemeinsam fördert.

10. Vorrichtung (100) nach einem der Ansprüche 3 bis 9, die so ausgebildet ist, dass der Behälter (30) parallel zu einer Längsachse (14) des mindestens einen Elektronenstrahlerzeugers (10) bewegbar ist.

11. Vorrichtung (100) nach einem der Ansprüche 1 bis 9, bei der eine Spannung, eine Elektronenenergie und/oder ein Magnetfeld des mindestens einen Elektronenstrahlerzeugers (10) variierbar ist, um unterschiedliche Zonen einer Innenseite des Behälters (30) mit dem Elektronenstrahlbündel (12) zu bestrahlen.

12. Vorrichtung (100) nach einem der Ansprüche 7 bis 9, bei der ein Strom des fremdinitiierten Elektromagneten variierbar ist, um unterschiedliche Zonen einer Innenseite des Behälters (30) mit dem Elektronenstrahlbündel (12) zu bestrahlen.

13. Verfahren zum Sterilisieren eines Behälters (30), mit den folgenden Schritten:

    Erzeugen eines Elektronenstrahlbündels (12) mittels eines Elektronenstrahlerzeugers (10), Fokussieren des Elektronenstrahlbündels (12), so dass das Elektronenstrahlbündel (12) fokussiert durch eine Behälteröffnung (32) in den Behälter (30) eintritt.

14. Verfahren nach Anspruch 13, bei dem das Fokussieren derart ausgeführt wird, dass sich das Strahlenbündel (12) jenseits eines Fokus der Fokussiereinrichtung (20) wieder auffächert und auf direktem Weg in dem gesamten Behälter (30) verteilt.

15. Verfahren nach Anspruch 13 oder 14, bei dem des weiteren ein Schritt des Förderns des Behälters (30) mittels einer Fördereinrichtung (40) vorgesehen ist, wobei die Fokussiereinrichtung (20) mit dem Behälter (30) mitbewegt wird und der Elektronenstrahlerzeuger (10) ortsfest verbleibt.

16. Verfahren nach Anspruch 13 oder 14, bei dem des weiteren ein Schritt des Förderns des Behälters (30) mittels einer Fördereinrichtung (40) vorgesehen ist, wobei der Elektronenstrahlerzeuger (10) und die Fokussiereinrichtung (20) mit dem Behälter (30) mitbewegt werden.

17. Verfahren nach einem der Ansprüche 12 bis 14, bei dem der Behälter (30) parallel zu einer Längsachse (14) des Elektronenstrahlerzeugers (10) bewegt wird, während das Elektronenstrahlbündel (12) in den Behälter (30) eintritt.

18. Verfahren nach einem der Ansprüche 13 bis 16, bei dem eine Spannung, eine Elektronenenergie und/oder ein Magnetfeld des Elektronenstrahlerzeugers (10) variiert wird, um unterschiedliche Zonen einer Innenseite des Behälters (30) mit dem Elektronenstrahlbündel (12) zu bestrahlen.

**19.** Verfahren nach einem der Ansprüche 13 bis 16 bei dem ein Strom der Fokussiereinrichtung (20) variiert wird, um unterschiedliche Zonen einer Innenseite des Behälters (30) mit dem Elektronenstrahlbündel (12) zu bestrahlen.

Fig. 1

Fig. 2

Fig. 3

401 — 400

f 1

Fig. 4

501 — 500

f 2

Fig. 5

Fig. 6

Fig. 7

**EP 2 218 465 A1**

| | Europäisches Patentamt / European Patent Office / Office européen des brevets | **EUROPÄISCHER RECHERCHENBERICHT** | **Nummer der Anmeldung**<br>EP 10 00 0337 |
|---|---|---|---|

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 1 956 608 A1 (JAPAN AE POWER SYSTEMS CORP [JP]) 13. August 2008 (2008-08-13) * Absätze [0001], [00 7], [00 8], [0 17], [ 177], [ 178]; Ansprüche; Abbildungen 18a, 15a, 20a, 20b; Beispiele * ----- | 1-19 | INV.<br>A61L2/08<br>B65B55/08 |
| X | WO 2007/095205 A2 (ADVANCED ELECTRON BEAMS INC [US]; AVNERY TZVI [US]) 23. August 2007 (2007-08-23) * Seite 2, Zeile 30 - Seite 3, Zeile 25; Ansprüche; Abbildungen; Beispiele * * Seite 5, Zeilen 3-21 * * Seite 8, Zeile 9 - Seite 9, Zeile 8 * ----- | 1-3, 10-11, 13-14,17 | |
| X | JP 2002 308229 A (ISHIKAWAJIMA HARIMA HEAVY IND) 23. Oktober 2002 (2002-10-23) * Zusammenfassung; Abbildungen * ----- | 1-3, 13-14 | |
| A | US 2007/283667 A1 (KRISTIANSSON ANDERS [SE] ET AL KRISTIANSSON ANDERS [SE] ET AL) 13. Dezember 2007 (2007-12-13) * das ganze Dokument * ----- | 1-19 | RECHERCHIERTE SACHGEBIETE (IPC)<br><br>A61L<br>B65B |

Der vorliegende Recherchenbericht wurde für alle Patentansprüche erstellt

| Recherchenort | Abschlußdatum der Recherche | Prüfer |
|---|---|---|
| München | 23. März 2010 | Nissen, Vagn |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

................................................................

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 10 00 0337

In diesem Anhang sind die Mitglieder der Patentfamilien der im obengenannten europäischen Recherchenbericht angeführten
Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

23-03-2010

| Im Recherchenbericht angeführtes Patentdokument | Datum der Veröffentlichung | Mitglied(er) der Patentfamilie | Datum der Veröffentlichung |
|---|---|---|---|
| EP 1956608 A1 | 13-08-2008 | WO 2007046213 A1<br>KR 20080014849 A<br>US 2009134338 A1 | 26-04-2007<br>14-02-2008<br>28-05-2009 |
| WO 2007095205 A2 | 23-08-2007 | CN 101416255 A<br>EP 1991993 A2<br>JP 2009526971 T<br>US 2008073549 A1 | 22-04-2009<br>19-11-2008<br>23-07-2009<br>27-03-2008 |
| JP 2002308229 A | 23-10-2002 | KEINE | |
| US 2007283667 A1 | 13-12-2007 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 2227059 C3

- US 20070237672 A1